# EUROPEAN PATENT SPECIFICATION

(11) **EP 2 661 262 B1**
(45) Date of publication and mention of the grant of the patent: **15.11.2017**
(21) Application number: 12706949.0
(22) Date of filing: 06.01.2012
(51) Int. Cl.: A61K 9/20, A61K 31/663, A61K 31/675, A61K 9/46

(54) **IMPROVED BISPHOSPHONATE FORMULATIONS**
VERBESSERTE BISPHOSPHONAT-FORMULIERUNGEN
FORMULATIONS AMÉLIORÉES DE BISPHOSPHONATES

(30) Priority: 06.01.2011 TR 201100149; 06.01.2011 TR 201100153
(43) Date of publication of application: 13.11.2013
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2012/000005
(87) International publication number: WO 2012/093974

(56) References cited:
- WO-A1-2010/090614
- WO-A1-2012/093974
- WO-A2-2011/146030
- AU-A4- 2007 101 090
- AU-A4- 2007 101 091
- AU-B2- 2001 268 722
- US-A1- 2004 121 007

## Description

### Technical Field

The present invention relates to stabile and highly bioavailable formulations comprising an active agent from bisphosphonate group for prophylaxis and treatment of bone loss and related diseases, and their production methods.

### The Prior Art

Bone loss or in other words "osteoporosis" is defined as a disease which is characterized by tendency for bone brittleness and increase in fracture risk caused by low bone mass and deformation of the micro structure of the bone tissue. Although this disease commonly does not result in death, it is a major health problem affecting life quality all over the world.

Bisphosphonate is a medicine group which is widely used for preventing and treating bone loss and related diseases. Research conducted shows that starting bisphosphonate treatment in the postmenopausal period can reduce fracture risk in presence of osteoporosis or non-traumatic bone fractures *(Fink HA the Fracture Intervention Trial (FIT) 2003).* Alendronic acid, risedronic acid and ibandronic acid and their derivatives are widely used active substances from the bisphosphonate group.

Risedronic acid, chemical name of which is [1-hydroxy-2-(3-pyridinyl) ethylidene] bisphosphonic acid, is a bisphosphonate *(Formula I).* It was first disclosed in the patent numbered US 5583122 and said patent comprises processes for preparing risedronic acid and its use in treatment of diseases related to abnormal calcium and phosphate metabolism.

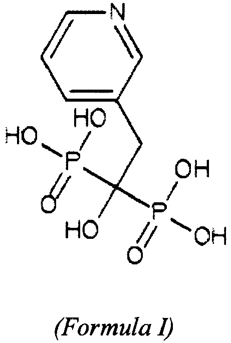

Alendronic acid, chemical name of which is (4-amino-1-hydroxybutylidene) bisphosphonic acid, is a bisphosphonate (Formula II).

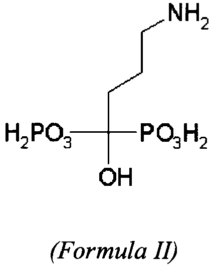

Alendronic acid was first disclosed in the patent numbered US4705651. Also, processes for preparation of alendronic acid and its use in the treatment of osteopathy have been described in this patent.

Active substances from bisphosphonate group which are used for preventing and treating bone loss and related diseases should be administered for long periods of time for an efficient treatment. It has been seen that oral administration of these active substances which are used for treating these kinds of diseases is advantageous for patients' adaptation to the treatment and continuity of the treatment.

However; in the case that formulations are administrated by the oral route, problems of low absorption of the active substance can be encountered. Absorption of active substances from bisphosphonate group decreases by 55% according to hungriness when taken half an hour before breakfast or 2 hours after dinner. It has been designated that when the active agents are given one hour before breakfast, their efficiency is preserved but the absorption is decreased by %30 according to hungriness even in this situation. On the other hand, in oral administration of bisphosphonates, irritations occur in gastrointestinal system in the case that the patient takes medicine with inadequate amount of water or does not remain in upright position for at least half an hour after the intake of the drug.

Another problem about bisphosphonate formulations is that the formulations comprising these active substances are affected by environmental conditions such as temperature and moisture and therefore they cannot remain stable for long periods of time under storage conditions.

Patent numbered EP1790347 claims that it solves this problem with formulations produced by wet granulation method and said formulations can remain stable for a long time without being affected by temperature and moisture conditions. The characteristic feature of the formulations in this patent is that a disintegrant is added to said formulations along with the active substance and the filling agent at the granulation step and the formulation prepared according to this method is wet-granulated.

The patent numbered US6294196, on the other hand, discloses formulations of diphosphonic acid derivatives including risedronate and alendronate designed for providing the desired effect. Formulations of the invention comprise 5% of lubricant by weight and are produced by wet granulation method.

The patent numbered WO 2000/021541 relates to administration of bisphosphonates in high doses. Dosage forms of the patent comprising high doses of bisphosphonate are prepared as granulated with a granulation solution comprising lactose, microcrystalline cellulose or polyvinylpyrrolidone. Other prior art documents are AU 2007101091 A4, WO 2010/090614 A1 and US 2004/0121007 A1. However during wet granulation of the bisphosphonates, there is a danger that active substances can form a complex with metal parts of the equipment used in granulation (invaluable metal ions) in an aqueous medium. The fact that the active agent forms a complex causes impurity in the active agent. Thus, this is an unfavorable situation for pharmaceutical technology.

When the prior art is taken into consideration, it is seen that there is need for new and easily producible bisphosphonate formulations that can provide high bioavailability, remain stable under storage conditions after production and also preserve purity of the active substance.

As a result of the studies conducted, the inventor has found out that these problems can be overcome with the help of components of the formulation and selecting a proper production method.

### Description of the Invention

The present invention relates to highly bioavailable new formulations comprising an active substance from bisphosphonate group and remaining stable for long periods of time.

The characteristic of the formulations according to the invention is that said formulations are prepared by a method characterized in that; (a) said method is a wet granulation method, (b) granulation solution of said method comprises maltodextrin and sorbitol wherein the ratio of maltodextrin to sorbitol in the granulation solution is in the range of 0.5 to 5 by weight. The inventor has surprisingly found out that the content of the granulation solution used in the production of the formulations comprising an active substance from bisphosphonate group and at least one pharmaceutically acceptable excipient by wet granulation method has an important effect on the stability and product purity of the final product formulations. Accordingly, the dosage forms prepared by wet granulation of the formulations comprising one active substance from bisphosphonate group and at least one pharmaceutically acceptable excipient with a granulation solution existing in the prior art which comprises cellulose derivatives like polyvinylpyrrolidone, microcrystalline cellulose and binders like starch, lactose and so forth do not remain stable under storage conditions for sufficient time. On the other hand, these dosage forms could not been produced with sufficient purity since the active agents complex with the metal parts of the equipment.

On the contrary, the studies conducted within the scope of the invention have surprisingly pointed out that formulations which have the highest stability and product purity are obtained by wet granulation method which utilizes a granulation solution comprising maltodextrin and sorbitol.

In other words, the characteristic of the method that shall be used for production of formulations of the invention and comprising one active substance from bisphosphonate group and at least one pharmaceutically acceptable excipient is that;
(a) It is a wet granulation method,
(b) The granulation solution comprises a combination of maltodextrin and sorbitol, wherein the ratio of maltodextrin to sorbitol in the granulation solution is in the range of 0.5 to 5 by weight.

Another characteristic of the method used for production of the formulations of the invention is that maltodextrin: sorbitol ratio is in the range of 1 to 4.5 by weight in the maltodextrin and sorbitol mixture forming the granulation solution.

Another characteristic of the method used for production of the formulations of the invention is that maltodextrin: sorbitol ratio is in the range of 1.3 to 4.5 by weight in the maltodextrin and sorbitol mixture forming the granulation solution.

Another characteristic feature of the method used for production of the formulations of the invention which comprise an active substance from the bisphosphonate group and at least one pharmaceutically acceptable excipient is composed of the following steps:
1. Preparing the active substance mixture by mixing the active substance and at least one pharmaceutically acceptable excipient,
2. Wet granulation of the active substance mixture obtained at the first step with the granulation solution comprising maltodextrin and sorbitol, wherein the ratio of maltodextrin to sorbitol in the granulation solution is in the range of 0.5 to 5 by weight,
3. Drying and sieving the granules,
4. Optionally adding at least one other pharmaceutically acceptable excipient to the obtained granules,
5. Giving the desired shape to the final granules.

In the production of the formulations according to the invention, granulation process can be performed twice successively. The granulation solution that can be used for granulating the formulations twice can comprise at least one pharmaceutically acceptable binder.

Another characteristic of the formulations of the invention which comprise an active agent from bisphosphonate group and at least one pharmaceutically acceptable excipient is that said formulations are produced by a method comprising the steps below;
1. Preparing the active substance mixture by mixing the active substance and at least one pharmaceutically acceptable excipient,
2. Wet granulation of the active substance mixture obtained at the first step with the granulation solution comprising maltodextrin and sorbitol, wherein the ratio of maltodextrin to sorbitol in the granulation solution is in the range of 0.5 to 5 by weight,
3. Optionally adding at least one other pharmaceutically acceptable excipient to the obtained granules and also optionally granulating the obtained composition with a proper granulation solution the second time,
4. Drying and sieving the granules,
5. Adding at least one pharmaceutically acceptable excipient to the obtained dry granules,
6. Giving the desired shape to the final granules.

In other words, another characteristic of the formulations of the invention is that said formulations comprising an active substance from bisphosphonate group and at least one pharmaceutically acceptable excipient are obtained by granulating them twice with a granulation solution comprising maltodextrin and sorbitol, wherein the ratio of maltodextrin to sorbitol in the granulation solution is in the range of 0.5 to 5 by weight.

Optionally, at least 30%, preferably 30-60% of the first granulation solution comprising sorbitol and maltodextrin, wherein the ratio of maltodextrin to sorbitol in the granulation solution is in the range of 0.5 to 5 by weight, can be used as the second granulation solution.

A characteristic of the formulations of the invention is that the active substance from bisphosphonate group used in the formulations is risedronate or alendronate or a pharmaceutical derivative thereof.

Risedronate used in the formulations of the invention can be in the form of its pharmaceutically acceptable hydrates, solvates, esters, enantiomers, polymorphs, crystalline forms, amorphous forms, salt or freebase form and/or a combination thereof. Risedronate that is contained in the formulation of the present invention is preferably in salt form. These salts can be selected from a group comprising sodium, potassium, calcium, magnesium and ammonium salts though sodium salt is preferred for the formulation of the invention. Risedronate which is comprised in the formulation of the present invention is in hydrate form. These hydrate forms can be selected from monohydrate, dihydrate, trihydrate, hemihydrates, ¼ hydrate, ⅓ hydrate, ²/₃ hydrate, ¾ hydrate, ⁵/₄ hydrate, ⁴/₃ hydrate, ⁵/₂ hydrate and ³/₂ hydrate. Risedronate which is comprised in the formulation of the present invention is preferably monohydrate or ⁵/₂ hydrate.

The formulations of the present invention can comprise risedronate in the range of 0,1-50% by weight. The amount of risedronate that the formulation of the present invention can comprise is in the range of 5-500 mg, preferably in the range of 10-200 mg.

Alendronate that the effervescent formulation of the present invention comprises can be in the form of its pharmaceutically acceptable hydrates, solvates, esters, enantiomers, polymorphs, crystalline forms, amorphous forms, salts or free base form and/or a combination thereof. Alendronate which is comprised in the effervescent formulation of the invention is preferably in salt form.

These salts can be selected from a group comprising organic acid salts like hydrochloride, hydrabromide, sulphate, phosphate, formate, acetate, trifluoroacetate, methanesulphonate, benzenesulphonate, toluenesulfonate; alkali metal salts like sodium, potassium; alkaline earth salts like calcium, magnesium; organic amine salts like trimethylamine, triethylamine, pyridine, picoline, dicyclohexylamine or a combination thereof.

Alendronate which is comprised in the effervescent formulation of the present invention is in sodium form.

Alendronate sodium which is comprised in the effervescent formulation of the present invention is preferably in hydrate form.

These hydrates can be selected from a group comprising hydrates like monohydrate, dihydrate, trihydrate, hemihydrate, ¹/₄ hydrate, ⅓ hydrate, ²/₃ hydrate, ¾ hydrate, ⁵/₄ hydrate, ⁴/₃ hydrate, ⁵/₂ hydrate and ³/₂ hydrate.

Alendronate sodium which is comprised in the effervescent formulation of the present invention is preferably in trihydrate form.

Alendronate which is comprised in the effervescent formulation of the present invention is alendronate sodium trihydrate.

The amount of alendronate that the effervescent formulation of the invention comprises is in the range of 1-200 mg, preferably in the range of 5-200 mg.

The amount of alendronate that the effervescent formulation of the invention comprises is in the range of 0,1-30, preferably in the range of 1-20% by weight.

The effervescent formulation of the invention can comprise pharmaceutically acceptable excipients selected from a group comprising binder, effervescent couple, lubricant, glidant, diluent, flavoring agent, sweetener, disintegrant, coloring agent, alkaline agent, surfactant, antifoam agent, viscosity agent, stabilizing agent or a combination thereof.

The alkaline agent that can be used in the formulations of the invention can be selected from a group comprising potassium carbonate, potassium bicarbonate, potassium citrate, potassium hydroxide, potassium tartrate, potassium phosphate, sodium carbonate, sodium bicarbonate, sodium citrate, sodium hydroxide, sodium tartrate, sodium phosphate and/or a combination thereof.

The binder that can be used in the formulations of the invention can be selected from a group comprising starches such as potato starch, corn starch, wheat starch; sugars such as sucrose, glucose, dextrose, lactose, maltodextrin; natural or synthetic gums; gelatin; cellulose derivatives such as microcrystalline cellulose, HPC, HEC, HPMC, carboxymethyl cellulose, methyl cellulose, ethyl cellulose; polyvinylpyrrolidone (povidone); polyethylene glycol (PEG); waxes; calcium carbonate; calcium phosphate; alcohols such as sorbitol, xylitol, mannitol and water or a combination thereof.

The lubricant that can be used in the formulations of the invention can be selected from a group comprising sodium lauryl sulphate, sodium benzoate, sodium chloride, sodium acetate, sodium fumarate, carbowax 4000, L-leucine (17), polyethylene glycol (PEG) or a combination thereof.

The lubricant that can be used in the formulations of the invention is polyethylene glycol (PEG). The amount of lubricant used in the formulation is in the range of 0,1-5%.

The glidant that can be used in the formulations of the invention can be selected from a group comprising talc, magnesium stearate, stearic acid, sodium stearyl fumarate, polyoxyethylene glycol, leucine, alanine, glycine, sodium benzoate, sodium acetate, fumaric acid or a combination thereof.

The diluent that can be used in the formulations of the invention can be selected from a group comprising lactose, maltose, dextrin, maltodextrin, mannitol, sorbitol, starch or a combination thereof.

The disintegrant that can be used in the formulations of the invention can be selected from a group comprising starches such as potato starch, corn starch, wheat starch, pregelatinized starch, sodium starch glycolate; cellulose derivatives such as croscarmellose sodium or microcrystalline cellulose; polyvinylpyrrolidone; crospovidone; alginic acid and its salts; clays like xanthan gum or veegum; ion exchange resin or a combination thereof.

However, the inventors have found out that desired dissolution is provided in the formulations of the invention without need for a disintegrant.

It is advantageous not to use a disintegrant in the formulations of the invention, as it inhibits a possible excipient - active substance interaction in the formulations.

The flavoring agent that can be used in the formulations of the invention can be selected from a group comprising natural aroma oils (such as peppermint oil, wintergreen oil, clove oil, parsley oil, eucalyptus oil, lemon oil, orange oil), menthol, menthane, anethole, methyl salicylate, eucalyptol, cinnamon, 1-methyl acetate, sage, eugenol, oxanone, alpha irisone, marjoram, lemon, orange, blackberry, propenyl guaethol acetyl, cinnamon, vanillin, thymol, linalol, cinnamaldehyde glycerol acetal, N-substituted p-menthane carboxamide, 3 1-methoxy propane 1, 2-diol or a combination thereof.

The sweetener that can be used in the formulations of the invention can be selected from a group comprising sucralose, sucrose, fructose, glucose, galactose, xylose, dextrose, laevulose, lactose, maltose, maltodextrin, mannitol, maltitol, maltol, sorbitol, xylitol, erythritol, lactitol, isomalt, corn syrup, saccharine, saccharine salts, acesulfame potassium, aspartame, D-tryptophane, monoammonium glycyrrhizinate, neohesperidin dihydrochalcone, thaumatine, neotame, alitame, stevioside and cyclamate or a combination thereof.

The coloring agent that can be used in the formulations of the invention can be selected from carotinoides and chlorophyll or a combination thereof.

The surfactant that can be used in the formulations of the invention can be selected from sodium lauryl sulphate and magnesium lauryl sulphate or a combination thereof.

The antifoam agent that can be used in the formulations of the invention can be selected from simethicone emulsion and dimethyl siloxane, silicon oil or a combination thereof.

The viscosity agent that can be used in the formulations of the invention can be selected from carboxymethyl cellulose, methyl cellulose, xanthan gum, gummi tragacanthae, gum arabic, aerosil 200, collodion, agar agar, bentonite, hydroxyethyl cellulose or a combination thereof.

The stabilizing agent and/or agents that can be used in the formulations of the invention can be selected from a group of agents comprising antioxidants, chelating agents, alkalinizing agents and photoprotectors.

The antioxidants that can be used in the formulations of the invention can be selected from a group comprising substances such as butylated hydroxyanisole (BHA), sodium ascorbate, butylated hydroxytoluene (BHT), sodium sulphite, gallates (such as propyl gallate), tocopherol, citric acid, malic acid, ascorbic acid, acetylcysteine fumaric acid, lecithin, ascorbyl palmitate, ethylenediamine tetraacetate or a combination thereof.

The chelating agents that can be used in the formulations of the invention can be selected from a group comprising disodium EDTA, edetic acid, citric acid, sodium citrate, potassium citrate or a combination thereof.

The alkalinizing agents that can be used in the formulations of the invention can be selected from a group comprising alkali metal salts such as sodium carbonate, sodium hydrogen carbonate, sodium hydroxide, sodium silicate, disodium hydrogen orthophosphate, sodium aluminate; alkaline earth metal salts such as calcium carbonate, calcium hydroxide, dibasic calcium phosphate, tribasic calcium phosphate, calcium sulphate, calcium acetate, calcium gluconate, calcium glycerophosphate, magnesium carbonate, magnesium hydroxide, magnesium sulphate, magnesium acetate, magnesium silicate, magnesium aluminate and organic compounds such as primary, secondary and tertiary amines, cyclic amines, N,N'-dibenzyl ethylenediamine, diethanolamine, ethylenediamine, meglumine, monosodium glutamate, polacrilin sodium, sodium alginate or a combination thereof.

The photoprotecting agents that can be used in the formulations of the invention can be selected from a group comprising metal oxides like titanium oxide, iron oxide or zinc oxide or a combination thereof.

The term "effervescent couple" mentioned throughout the invention refers to use of one acidic and one basic agent together.

The acidic agents that can be used in the formulations of the invention can be selected from a group comprising other organic acids such as citric and acetic acid, tartaric acid, fumaric acid, adipic acid, malic acid or a combination thereof.

The basic agents that can be used in the formulations of the invention can be selected from a group comprising potassium carbonate, potassium bicarbonate, potassium citrate, potassium hydroxide, sodium carbonate and sodium hydrogen carbonate or a combination thereof.

Formulations of the present invention are administered by the oral route. Within this scope, said dosage forms administered by the oral route can be in solid forms such as pill, tablet, capsule, film tablet, orodispersible tablet, enterically coated tablet, modified-release tablet, prolonged-release tablet, delayed-release tablet, effervescent powder, effervescent granule, effervescent tablet, sachet, dragee, pastille or in liquid forms such as suspension, emulsion, syrup, drop, solution; preferably in solid form.

It has been seen that solid dosage forms are more advantageous in terms of ensuring precise and accurate dose adjustments within the scope of the present invention and longer shelf life. Bioavailability of bisphosphonates varies according to dosage form. For example, solid dosage forms like pill, tablet and capsules have to be broken into small pieces and dissolved in the stomach and small intestine in order to be absorbed. Bioavailability is delayed depending on this dispersion and dissolution processes. Furthermore, it is known that solid dosage forms like pill, tablet and capsule cause difficulty of use for geriatric, pediatric and disabled patients who have swallowing difficulties.

Depending on this, the patient delays the drug intake and this affects the efficiency of the treatment and life quality of the patient.

In the case that the inventors formulate the bisphosphonate formulations they have developed within the scope of the invention in effervescent powder, granule or tablet form; the drug is not exposed to dispersion or dissolution processes since it is taken as dissolved in water and therefore, bioavailability is not delayed.

On the other hand, the formulations prepared in this manner are also advantageous as they offer an easy-to-use formulation to the patients.

Effervescent formulations of the invention comprise at least one effervescent couple as excipient.

The amount of pharmaceutically acceptable acidic agent that can be used in the effervescent formulations of the invention is in the range of 40% - 70 % by weight and the amount of the pharmaceutically acceptable basic agent is in the range of 20% - 50% by weight. Effervescent formulations of the invention are in effervescent powder, effervescent granule or effervescent tablet form.

The effervescent powder, granule or tablet formulations of the invention are preferably produced by a method comprising the steps of;
1. Preparing the active agent mixture by mixing the active agent and the basic agent,
2. Wet-granulating the active agent mixture obtained in the first step with a granulation solution comprising maltodextrin and sorbitol mixture, wherein the ratio of maltodextrin to sorbitol in the granulation solution is in the range of 0.5 to 5 by weight,
3. Adding at least one pharmaceutically acceptable acidic agent and optionally another excipient to the granules and optionally granulating the obtained mixture for the second time with a proper granulation solution,
4. Drying and sieving the granules,
5. Adding at least one pharmaceutically acceptable flavoring agent to the obtained dry granules,
6. Giving the desired shape to the final granules.

Formulations of the invention can be combined with another active substance optionally.

The expression "other active substance" refers to various vitamins and/or minerals essential for human body.

As well as taken separately, simultaneously or sequentially for combined therapy, the dosage forms can also be taken by combining the active substance from bisphosphonate group with another active substance or substances in a single dosage form.

Other active substances that can be used together with the active substance from bisphosphonate group in combined therapy can be minerals such as calcium, potassium, magnesium, iron, sodium, zinc or their salts such as carbonate, sulphate; vitamin A, vitamin B's such as B₁, B₁₂, B₆ and/or folic acid, vitamin C, vitamin D and its analogues (such as vitamin D2 [ergocalciferol], vitamin D3 [colecalciferol]), vitamin E.

In combined therapy, one or two of the other active substances mentioned above can be combined with the active substance from bisphosphonate group.

In other words, the present invention comprises dual or triple combinations of the active substance from bisphosphonate group with above mentioned other active substances. The second active substances that can be used in the formulations of the invention are vitamin D and its analogues (such as vitamin D2 [ergocalciferol], vitamin D3 [colecalciferol]) and/or calcium.

Other active substance or substances that can be used in combined therapy can be produced together with bisphosphonate group active substance and by the same production method as well as prepared as a combined treatment package prepared by producing the active substance formulations separately and combining them afterwards.

Formulations of the invention can be used in treatment of osteoporosis; in osteoporosis treatment of women for reducing bone fracture risks including spine and hipbones in the postmenopausal period; in osteoporosis treatment of men for reducing bone fracture risks; treatment of diseases such as idiopathic osteoporosis, osteoporosis induced by various diseases, steroid and glucocorticoid-induced osteoporosis, osteopenia, osteomalacia, osteogenesis imperfecta, osteochondrodysplasia, Sudek's atrophy, rheumatoid arthritis, Paget's disease, metastases of malignant tumors to bone, hypocalcaemia or hyperthyroidism; and especially as a nutritional supplement for adolescent women, pregnant or breastfeeding women in pre and post-menopausal periods in order to maintain bone health.

In order to render the formulations of the present invention more comprehensible, essential examples are given below. Yet, the invention cannot be limited to these examples.

### EXAMPLES

### Example 1

| **Component** | **Amount (% by weight)** |
|---|---|
| Risedronat sodium | 2 |
| Sodium bicarbonate | 40 |
| Citric acid anhydrate | 50 |
| Polyethylene glycol | 2 |
| Maltodextrin | 3.50 |
| Sorbitol | 1.50 |
| **Total** | **100** |

Granulation solution is prepared by mixing sorbitol and maltodextrin. Risedronate and effervescent couple are mixed in fluid bed and granulated with the prepared granulation solution. Obtained granules are mixed with lubricant and the other excipients. Prepared composition is preferably compressed in tablet form.

### Example 2

| **Component** | **Amount (% by weight)** |
|---|---|
| Alendronate sodium trihydrate | 3.5 |
| Sodium bicarbonate | 30 |
| Sodium carbonate | 10 |
| Citric acid anhydrate | 48.5 |
| Polyethylene glycol | 1 |
| Maltodextrin | 3.5 |
| Sorbitol | 2 |
| Flavoring agent | 1.5 |
| **Total** | **100** |

Granulation solution is prepared by mixing sorbitol and maltodextrin. Alendronate, sodium carbonate and sodium bicarbonate are mixed in fluid bed and granulated with 50% of the prepared granulation solution. Effervescent acid and polyethylene glycol are added to the obtained granules and then granulated with the other 50% of the granulation solution. Flavoring agent is added to the obtained composition and this final composition is sieved. The final composition is preferably compressed in tablet form.

## Claims

1. A method for the production of formulations comprising one active agent from bisphosphonate group and at least one pharmaceutically acceptable excipient, **characterized in that**;
(a) said method is a wet granulation method,
(b) granulation solution of said method comprises maltodextrin and sorbitol wherein the ratio of maltodextrin to sorbitol in the granulation solution is in the range of 0.5 to 5 by weight.

2. The method according to claim 1 **characterized in that** the ratio of maltodextrin to sorbitol in the granulation solution of said method is in the range of 1 to 4.5 by weight.

3. The method according to claims 1-2 **characterized in that** the ratio of maltodextrin to sorbitol in the granulation solution of said method is in the range of 1.3 to 4.5 by weight.

4. The method according to any preceding claims **characterized in that** said method comprises the steps of,
• Preparing the active agent mixture by mixing the active agent and at least one pharmaceutically acceptable excipient,
• Wet granulation of the active agent mixture obtained in the first step with the granulation solution comprising maltodextrin and sorbitol,
• Drying and sieving the granules,
• Optionally, adding at least one other pharmaceutically acceptable excipient to the obtained granules,
• Giving the desired shape to the final granules.

5. The method according to claims 1-3 **characterized in that** said method comprises the steps of,
• Preparing the active agent mixture by mixing the active agent and at least one pharmaceutically acceptable excipient,
• Wet granulation of the active agent mixture obtained in the first step with the granulation solution comprising maltodextrin and sorbitol,
• Optionally, adding at least one other pharmaceutically acceptable excipient to the obtained granules and optionally, granulating the obtained composition with a proper granulation solution for the second time.
• Drying and sieving the granules,
• Optionally, adding at least one other pharmaceutically acceptable excipient to the obtained granules,
• Giving the desired shape to the final granules.

6. The method according to claim 5 **characterized in that** at least 30% of the first granulation solution comprising sorbitol and maltodextrin is used as the second granulation solution.

7. The method according to claim 6 **characterized in that** at least 30-60% of the first granulation solution comprising sorbitol and maltodextrin is used as the second granulation solution.

8. The method according to claim 1 **characterized in that** the active agent from bisphosphonate group is alendronate or risedronate or a pharmaceutically acceptable derivative thereof.

9. A formulation obtained by a method according to claim 1, and comprising one active agent from the bisphosphonate group and at least one pharmaceutically acceptable excipient, **characterized in that** the active agent from the bisphosphonate group is alendronate or risedronate wherein the formulation comprises maltodextrin and sorbitol, and wherein the ratio of maltodextrin to sorbitol in the formulation is in the range of 0.5 to 5 by weight.

10. The formulation according to claim 9 **characterized in that** said formulation comprises risedronate in the range of 0,1-50% by weight, or, wherein said formulation comprises alendronate in the range of 0,1-30% by weight.

11. The formulation according to claim 10, wherein said formulation comprises risedronate, **characterized in that** risedronate is in the form of its pharmaceutically acceptable hydrates, solvates, esters, enantiomers, polymorphs, crystalline forms, amorphous forms, salt forms or freebase form and/or a combination thereof.

12. The formulation according to claim 10, wherein said formulation comprises alendronate, characterized alendronate is in the form of its pharmaceutically acceptable hydrates, solvates, esters, enantiomers, polymorphs, crystalline forms, amorphous forms, salt forms or freebase form and/or a combination thereof.

13. The formulation according to claims 10 and 12 **characterized in that** said formulation comprises at least one excipient selected from a group comprising binder, effervescent couple, lubricant, glidant, diluent, flavoring agent, sweetener, disintegrant, coloring agent, alkali agent, surfactant, antifoam agent, viscosity agent, stabilizing agent or a combination thereof.

14. The formulation according to claims 10 and 12 **characterized in that** said formulations are formulated in any solid form such as pill, tablet, capsule, film tablet, orodispersible tablet, enterically coated tablet, modified-release tablet, prolonged-release tablet, delayed-release tablet, effervescent powder, effervescent granule, effervescent tablet, sachet, dragee or pastille.

15. The formulation according to claim 14 **characterized in that** said formulations are effervescent powder, granule or tablet form.

## Patentansprüche

1. Eine Methode für
die Produktion von Formulierungen bestehend aus einem aktiven Mittel von Bisphosphonatgruppe und mindestens einem pharmazeutisch annehmbaren Arzneistoffträger, **dadurch gekennzeichnet, daß**;
(a) besagte Methode eine Feuchtgranulationsmethode ist,
(b) Granulationslösung der besagten Methode Maltodextrin und Sorbitol beinhaltet, wo das Verhältnis von Maltodextrin zum Sorbitol in Granulationslösung im Bereich von 0.5 bis 5 nach Gewicht beträgt.

2. Die Methode nach Anspruch 1, **dadurch gekennzeichnet, daß** das Verhältnis von Maltodextrin zum Sorbitol in Granulationslösung der besagten Methode im Bereich von 1 bis 4.5 nach Gewicht beträgt.

3. Die Methode nach Ansprüche 1 bis 2, **dadurch gekennzeichnet, daß** das Verhältnis von Maltodextrin zum Sorbitol in Granulationslösung der besagten Methode im Bereich von 1.3 bis 4.5 nach Gewicht beträgt.

4. Die Methode nach den vorhergehenden Ansprüche, **dadurch gekennzeichnet, daß** besagte Methode besteht aus folgenden Schritten,
• Vorbereitung der aktiven Mittelmischung durch Einmischung von aktivem Mittel und mindestens einem pharmazeutisch annehmbaren Arzneistoffträger,
• Feuchtgranulation der aktiven Mittelmischung erhalten im ersten Schritt mit Granulationslösung, die Maltodextrin und Sorbitol beinhaltet,
• Trocknung und Siebung der Körner,
• Optional, Hinzufügung von mindestens einem anderen pharmazeutisch annehmbaren Arzneistoffträger zur erhaltenen Körner,
• Eingabe der gewünschten Form zu den Schlusskörnern.

5. Die Methode nach Ansprüche 1 bis 3, **dadurch gekennzeichnet, daß** besagte Methode besteht aus den Schritten,
• Vorbereitung der aktiven Mittelmischung durch Einmischung von aktivem Mittel und mindestens einem pharmazeutisch annehmbaren Arzneistoffträger,
• Feuchtgranulation der aktiven Mittelmischung erhalten im ersten Schritt mit Granulationslösung, die Maltodextrin und Sorbitol beinhaltet,
• Optional, Hinzufügung von mindestens einem anderen pharmazeutisch annehmbaren Arzneistoffträger zur erhaltenen Körner und optional, Granulation der erhaltenen Komposition mit einer geeigneten Granulationslösung für das zweite Mal.
• Trocknung und Siebung der Körner,
• Optional, Hinzufügung von mindestens einem anderen pharmazeutisch annehmbaren Arzneistoffträger zur erhaltenen Körner,
• Eingabe der gewünschten Form zu den Schlusskörnern.

6. Die Methode nach Anspruch 5, **dadurch gekennzeichnet, daß** mindestens 30 % der ersten Granulationslösung beinhaltet Sorbitol und Maltodextrin als zweite Granulationslösung verwendet wird.

7. Die Methode nach Anspruch 6, **dadurch gekennzeichnet, daß** mindestens 30 - 60 % der ersten Granulationslösung beinhaltet Sorbitol und Maltodextrin als zweite Granulationslösung verwendet wird.

8. Die Methode nach Anspruch 1, **dadurch gekennzeichnet, daß** aktives Mittel von Bisphosphonatgruppe Alendronate oder Risedronate oder ein pharmazeutisch annehmbares Derivat von denen ist.

9. Eine Formulierung erhalten mit einer Methode nach Anspruch 1, und beinhaltet ein aktives Mittel von Bisphosphonatgruppe und mindestens einen pharmazeutisch annehmbaren Arzneistoffträger, **dadurch gekennzeichnet, daß** das aktive Mittel von Bisphosphonatgruppe Alendronate oder Risedronate ist, wo die Formulierung beinhaltet Maltodextrin und Sorbitol, und wo das Verhältnis von Maltodextrin zum Sorbitol in Formulierung im Bereich von 0.5 bis 5 % nach Gewicht beträgt.

10. Die Formulierung nach Anspruch 9, **dadurch gekennzeichnet, daß** besagte Formulierung beinhaltet Risedronate im Bereich von 0,1-50% nach Gewicht, oder, wo besagte Formulierung beinhaltet Alendronate im Bereich von 0,1-30% nach Gewicht.

11. Die Formulierung nach Anspruch 10, wo besagte Formulierung beinhaltet Risedronate, **dadurch gekennzeichnet, daß** Risedronate in der Form ist von seiner pharmazeutisch annehmbaren Hydrate, Solvate, Ester, Enantiomer, Polymorphs, Kristallformen, gestaltlose Formen, Salzformen oder freie Basenform und/oder eine Kombination von denen.

12. Die Formulierung nach Anspruch 10, wo besagte Formulierung beinhaltet Alendronate, **dadurch gekennzeichnet, daß** Alendronate in der Form ist von seiner pharmazeutisch annehmbaren Hydrate, Solvate, Ester, Enantiomer, Polymorphs, Kristallformen, gestaltlose Formen, Salzformen oder freie Basenform und/oder eine Kombination von denen.

13. Die Formulierung nach Anspruch 10 und 12, **dadurch gekennzeichnet, daß** besagte Formulierung beinhaltet mindestens einen Arzneistoffträger ausgewählt von einer Gruppe bestehend aus Bindemittel, übersprudelndes Paar, Schmiermittel, Glidant, Verdünnungsmittel, Aromastoff, Süßstoff, Disintegrin, Farbstoff, Alkalimittel, Benetzungsmittel, Antischaummittel, Viskositätsmittel, Stabilisierungsmittel oder eine Kombination von denen.

14. Die Formulierung nach Ansprüche 10 und 12, **dadurch gekennzeichnet, daß** besagte Formulierungen formuliert sind in fester Form wie Pille, Tablette, Kapsel, Filmtablette, dispergierbare Tablette, magensaftresistente Tablette, modifiziert-release Tablette, anhaltend-release Tablette, verzögert-release Tablette, übersprudelndes Pulver, übersprudelndes Granulat, übersprudelnde Tablette, Sachet, Dragee oder Pastille.

15. Die Formulierung nach Anspruch 14, **dadurch gekennzeichnet, daß** besagte Formulierungen übersprudelndes Pulver, Granulat- oder Tablettenform sind.

## Revendications

1. Procédé pour la production de formulations comprenant un agent actif du groupe bisphosphonate et au moins un excipient pharmaceutiquement acceptable, **caractérisé en ce que**;
(a) ledit procédé est un procédé de granulation humide,
(b) la solution de granulation dudit procédé comprend de la maltodextrine et du sorbitol dans laquelle le rapport de la maltodextrine au sorbitol dans la solution de granulation est dans la plage de 0,5 à 5 en poids.

2. Procédé selon la revendication 1, **caractérisé en ce que** le rapport de la maltodextrine au sorbitol dans la solution de granulation dudit procédé est dans la plage de 1 à 4,5 en poids.

3. Procédé selon les revendications 1 à 2, **caractérisé en ce que** le rapport de la maltodextrine au sorbitol dans la solution de granulation dudit procédé est dans la plage de 1,3 à 4,5 en poids.

4. Procédé selon l'une quelconque des revendications précédentes, **caractérisé en ce que** ledit procédé comprend les étapes de,
• Préparation du mélange d'agents actifs en mélangeant l'agent actif et au moins un excipient pharmaceutiquement acceptable,
• Granulation humide du mélange d'agent actif obtenu dans la première étape avec la solution de granulation comprenant de la maltodextrine et du sorbitol,
• Séchage et tamisage des granulés,
• Eventuellement, en ajoutant au moins un autre excipient pharmaceutiquement acceptable aux granules obtenus,
• Donner la forme désirée aux granules finaux.

5. Procédé selon les revendications 1 à 3, **caractérisé en ce que** ledit procédé comprend les étapes suivantes:
• Préparation du mélange d'agents actifs en mélangeant l'agent actif et au moins un excipient pharmaceutiquement acceptable,
• Granulation humide du mélange d'agent actif obtenu dans la première étape avec la solution de granulation comprenant de la maltodextrine et du sorbitol,
• Eventuellement, en ajoutant au moins un autre excipient pharmaceutiquement acceptable aux granulés obtenus et facultativement, en granulant la composition obtenue avec une solution de granulation appropriée pour la deuxième fois.
• Séchage et tamisage des granulés,
• Eventuellement, en ajoutant au moins un autre excipient pharmaceutiquement acceptable aux granules obtenus,
• Donner la forme désirée aux granules finaux.

6. Procédé selon la revendication 5, **caractérisé en ce qu'**au moins 30% de la première solution de granulation comprenant du sorbitol et de la maltodextrine est utilisée comme seconde solution de granulation.

7. Procédé selon la revendication 6, **caractérisé en ce qu'**au moins 30 à 60% de la première solution de granulation comprenant du sorbitol et de la maltodextrine est utilisée comme seconde solution de granulation.

8. Procédé selon la revendication 1, **caractérisé en ce que** l'agent actif du groupe bisphosphonate est l'alendronate ou le risedronate ou un dérivé pharmaceutiquement acceptable de celui-ci.

9. Formulation obtenue par un procédé selon la revendication 1, et comprenant un agent actif du groupe bisphosphonate et au moins un excipient pharmaceutiquement acceptable, **caractérisé en ce que** l'agent actif du groupe bisphosphonate est l'alendronate ou le risedronate dans lequel la formulation comprend de la maltodextrine et sorbitol, et dans lequel le rapport de la maltodextrine au sorbitol dans la formulation est dans la plage de 0,5 à 5 en poids.

10. Formulation selon la revendication 9, **caractérisée en ce que** ladite formulation comprend du risédronate dans la plage de 0,1 à 50% en poids, ou dans laquelle ladite formulation comprend de l'alendronate dans la plage de 0,1 à 30% en poids.

11. Formulation selon la revendication 10, dans laquelle ladite formulation comprend du risédronate, **caractérisé en ce que** le risédronate se présente sous la forme de ses hydrates, solvates, esters, énantiomères, polymorphes, formes cristallines, formes amorphes, formes salines ou forme libre et pharmaceutiquement acceptables, formes cristallines, combinaison de ceux-ci.

12. Formulation selon la revendication 10, dans laquelle ladite formulation comprend de l'alendronate, l'alendronate caractérisé sous forme de ses hydrates, solvates, esters, énantiomères, polymorphes, formes cristallines, formes amorphes, formes salines ou forme libre et pharmaceutiquement acceptables, formes cristallines, combinaison de ceux-ci.

13. Formulation selon les revendications 10 et 12, **caractérisée en ce que** ladite formulation comprend au moins un excipient choisi parmi un groupe comprenant un liant, un couple effervescent, un lubrifiant, un agent gluant, un diluant, un agent aromatisant, un édulcorant, un agent colorant, un agent alcalin, un agent tensioactif, un agent antimousse agent, agent de viscosité, agent stabilisant ou une combinaison de ceux-ci.

14. Formulation selon les revendications 10 et 12, **caractérisée en ce que** lesdites formulations sont formulées sous n'importe quelle forme solide telle qu'une pilule, une tablette, une capsule, un comprimé de film, un comprimé orodispersible, un comprimé enrobé entérique, un comprimé à libération modifiée, un comprimé à libération prolongée, une libération retardée comprimé, poudre effervescente, granulé effervescent, comprimé effervescent, sachet, dragée ou pastille.

15. Formulation selon la revendication 14, **caractérisée en ce que** lesdites formulations sont poudre effervescente, granule ou forme de comprimé.
